(19) Europäisches Patentamt / European Patent Office / Office européen des brevets

(11) **EP 3 957 244 A1**

(12) **EUROPEAN PATENT APPLICATION**

(43) Date of publication:
**23.02.2022 Bulletin 2022/08**

(21) Application number: **20191902.4**

(22) Date of filing: **20.08.2020**

(51) International Patent Classification (IPC):
**A61B 5/16** (2006.01)          **A61B 5/11** (2006.01)
**A61B 5/00** (2006.01)

(52) Cooperative Patent Classification (CPC):
**A61B 5/165; A61B 5/1118; A61B 5/4815;**
**A61B 5/7267; G16H 10/20; G16H 50/20;**
A61B 5/1123; A61B 5/681; A61B 5/6823;
A61B 2562/0219

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **MINDPAX s.r.o.**
**128 00 Praha 2 (CZ)**

(72) Inventor: **Novak,, Jan**
**156 00 Praha 5 (CZ)**

(74) Representative: **Lahrtz, Fritz**
**Patentanwälte**
**Isenbruck Bösl Hörschler PartG mbB**
**Prinzregentenstraße 68**
**81675 München (DE)**

(54) **METHOD FOR DETECTION OF A RELAPSE INTO A DEPRESSION OR MANIA STATE BASED ON ACTIVITY DATA AND/OR DATA OBTAINED BY QUESTIONING THE PATIENT**

(57) The invention relates to a method for detection of a relapse into a depression or mania state of a patient from a remission state wherein motor activity data is recorded using a wearable device worn by the patient and is received as input data by an evaluating unit and/or mood data is acquired by obtaining a questionnaire which has been completed by the patient, the questions of the questionnaire relating to the mania state, to the depression state and the questionnaire including at least one control question for checking the awareness and/or the ability to focus of the patient, the questions being designed such that they can be answered by multiple choice, and wherein the answers of the patient are input as input data into the evaluating unit, the input data is analyzed by the evaluating unit, wherein the condition of the patient is classified as remission, mania or depression by means of machine learning, and wherein a relapse is detected if the patient is classified as mania or depression.

Further aspects of the invention relate to an evaluating system for detection of a relapse into a depression or mania state of a patient in a remission state based on motor activity data and/or mood data.

**Description**

The invention relates to a method for detection of a relapse into a depression or mania state of a patient from a remission state wherein activity data is recorded using a wearable device worn by the patient and is received as input data by an evaluating unit and/or mood data is acquired by obtaining a questionnaire which has been completed by the patient and is then input as input data into the evaluating unit and the input data is analyzed.

**State of the art**

Bipolar Disorder (BD) is a severe and chronic mental illness. The main symptom of BD is recurrent changing of symptomatic episodes of depression or of elevated mood (mania) with non-symptomatic (remission) periods.

There have been many attempts to rate or quantify the level of depression or mania in order to optimize the treatment and to assess the response to treatment.

In order to assess the current condition of a patient it is state of the art to observe the patient and to make use of patient self-report tools. In such patient self-report tools, the condition is assessed based on the patient's subjective perspective by evaluating questionnaires which have been completed by the patient.

Further, it is known that the three possible episodes of BD depression (dep), mania (man), and remission (rem) are linked to different degrees and patterns of physical activity. Change in social rhythms, sleep disruption and proportional change in affective tuning (subjective mood) precedes the repeated outbreaks of bipolar disorder (BD).

In case of a relapse, i.e. a significant deterioration of clinical condition, hospitalization, or a sharp proportional alteration in the subjective mood, of BD, often a long-term hospitalization of the patient is necessary. This represents a huge burden for the patient and his/her closest relatives and also results in high costs for society. Consequently, the detection of changes in social rhythmicity is also important from the medical point of view.

EP3430978 discloses systems and methods for logging subjective and objective patient data. The data is used to detect mood swings and changes in social rhythmicity. The system comprises a wearable device, such as a wristwatch, which includes an accelerometer to monitor physical activity. Further, the system may comprise an input interface for collecting a personal parameter associated with a scale. It is envisioned to perform analysis of the data by means of artificial intelligence. However, the prior art is silent on how this may be achieved with the required accuracy and statistical significance.

It is an object of the present invention to provide methods and corresponding systems to detect a relapse of a patient into a depression or mania state.

**Disclosure of the invention**

A method for detection of a relapse into a depression or mania state of a patient from a remission state is provided, wherein i) motor activity data is recorded using a wearable device worn by the patient and is received as input data by an evaluating unit and/or ii) mood data is acquired by obtaining a questionnaire which has been completed by the patient, the questions of the questionnaire relating to the mania state, to the depression state and the questionnaire including at least one control question for checking the awareness and/or the ability to focus of the patient, , and wherein the answers of the patient are input as input data into the evaluating unit.

The input data is analyzed by the evaluating unit, wherein the condition of the patient is classified as remission, mania or depression by means of machine learning.

The patient is detected to experience a relapse if he/she is classified as mania or depression. Accordingly, it is preferred to perform an appropriate action. The action may be selected, for example, from triggering an alarm, providing information relating to the relapse to a counselling psychiatrist or adapting a treatment plan. If the patient is classified as remission, no further action is required.

An alarm may, for example, be sent by e-mail or any other communication means to the patient's attending physician, psychiatrist or any other relevant person. The alarm may include a call for therapeutic intervention.

In case a therapeutic intervention is needed, the physician may, after he/she may have discussed the possible consequences with the patient, take the appropriate measures depending on the disease. This may include the administration or the adjustment of the medication needed for treating the patient's disease, other measures like psychotherapy, light therapy, rTMS (Repetitive Transcranial Magnetic Stimulation) or physical therapy as well as surgery. Administration of medication may include antipsychotics and mood stabilizers e.g. based on lithium/valproate like lurasidone, olanzapine and quetiapine.

Preferably, the wearable device is configured as a wristband or wrist-watch. Alternatively, the wearable device may comprise means for being attached to a belt onto the arm or around the hips. The wearable device may, for example, use one or more accelerometers or similar devices to record motion and in a particular acceleration of the wearable device and thus of the patient.

Movement acceleration values, especially movement acceleration values from a range of 0 to 2 g, may be considered and collected as activity data that is related to bipolar disorders.

Preferably, the activity data is aggregated over a data aggregation time interval by assigning the largest magnitude or an average magnitude to the respective aggregation time interval.

The activity data may, for example, be measured by means of an accelerometer, wherein an acceleration value with the largest magnitude or an average value of all measured acceleration values is selected and stored for each data aggregation time interval.

The "data aggregation time interval" may be a fixed interval in the range from 0.01 s to 300 s, in particular from 0.1 s to 10 s, in particular from 0.1 s to 0.2 s.

The acquired activity data is analyzed utilizing machine learning methods. Preferably, activity data according to variant i) is analyzed using a logistic regression method utilizing a time series of activity data. Logistic regression is a statistical binary model which may be used to differentiate between two different classes.

The used logistic regression model(s) are preferably trained by means of a labeled training dataset. The labels may in particular be provided in the form of a clinical scale which may have been prepared by means of external evaluation by a psychiatrist.

Preferably, validation of the trained models is performed after training the respective model using the training dataset. This may be performed using a separate validation dataset or, especially in case only a limited amount of data is available for training and validation, by means of selected leave-one-out cross-validation.

As it is intended to differentiate between three classes, namely depression, mania and remission, more than one logistic regression model is preferably used and the results of the models are combined.

In the variant ii), it is preferred to use logistic mixed effects models for the analysis of a time series of mood data. Logistic mixed effects models may be used in binary classification problems and comprise fixed and random effects.

In order to assign values to the answers, it is preferred that the questions are designed such that they may be answered by means of multiple presented choices, wherein each of the choices is assigned a certain value, or in form of a scale of values.

As it is intended to differentiate between three classes, namely depression, mania and remission, it is likewise preferred to use more than one logistic mixed effects model and to combine the results for the classification of the patients.

In a first logistic mixed effects model describing the probability for the patient being in the state of remission or mania, the mania related questions are assigned a value and this value is included in the model. In a second logistic mixed effects model describing the probability for the patient being in the state of remission or depression, the depression related questions are assigned a value and this value included in the model.

Preferably, the machine learning model used according to variant i) comprises binary classification models for classification of remission/mania, depression/mania and remission/depression and for classification into one of the three classes remission, mania and depression.

In one embodiment, for combining the results of the separate binary classification models, a score is computed for each of the three classes based on probabilities obtained from the binary classification models.

For example, the scores for remission, mania and depression may be computed using the formulas

$$Man_{score} = p_{dep\text{-}man} + p_{rem\text{-}man},$$

$$Dep_{score} = 1 - p_{dep\text{-}man} + p_{rem\text{-}dep},$$

$$Rem_{score} = 1 - p_{rem\text{-}man} + 1 - p_{rem\text{-}dep},$$

wherein the probabilities p are values in the range from 0 to 1, wherein a value of $p_{rem\text{-}man} = 1$ and $p_{dep\text{-}man} = 1$, respectively, is equivalent to a 100% probability for mania, $p_{rem\text{-}man} = 0$ and $p_{rem\text{-}dep} = 0$, respectively, is equivalent to a 100% probability for remission and $p_{rem\text{-}dep} = 1$ and $p_{dep\text{-}man} = 0$, respectively, is equivalent to a 100% probability for depression.

In another embodiment, one of the three classes is selected based on majority voting where two of the three classification models indicate the same class.

The questionnaire of variant ii) may be presented to the patient using an electronic device or may be presented in paper form.

Preferably, the questionnaire is presented to the patient on a screen and the answers to the questions are received using an input device, which is in particular configured for button-input, touch input or voice input.

In a preferred embodiment of the method, which combines both variants i) and ii), the wearable device which is used to record activity data includes the screen and/or the input device. Alternatively, an app is configured to allow a smart device to function as the screen and the input means.

In the variant i), motor activity data is analyzed. Preferably, features are extracted from the time series of activity data and the extracted features are used in the logistic regression model. Preferably, the features are calculated for each day. The features are preferably being selected from sleep features, in particular sleep duration, activity during sleep and fragmentation of sleep, activity distribution within the day, in particular the amount of activity in the active part of a day, overall activity level, fragmentation of activity within a day, the timing of activities throughout a day and combinations of at least two of said features.

The most important features include sleep duration. Sleep may be detected using an algorithm based on different activity in segments of different length. Based on the nature of activity within these segments the sleep-like segments are divided into wear-off and sleep. Sum of periods of sleep in a day is one of the most important features. Another important feature is a motor activity during the wake hours, specifically in the 10 most active hours. The average of the activity counts within the 10-hour window for each day as well as the midtime of daily activity peaks.

Preferably, the time series of activity data according to variant i) is divided into epochs. Features are estimated either based on epochs spanning individual days or weeks or longer periods, such as for example two weeks or a month of activity. Segments of sleep and wear-offs are detected though all the data and only afterward aggregated into daily values. Preferably, features are estimated using midnight to midnight data and only features based on night sleep are using different divisions. When a window is used for feature estimation, than the windows are overlapped in such a way that there is one value for a day (7 day window - 6 day overlap).

For example when a single sample of motor activity data represents 30 seconds of activity data, an epoch has 2880 samples (1440 minutes, 24 hours) for day-based features and 20160 resp. 40320 samples long for 7 day resp. 14 day window based features.

The epochs begin at daybreak local time for day-based features and at 18 o'clock local for sleep parameters. When windows longer than one day are used, they are overlapped as described previously.

A feature may be extracted from the entire time series of activity data or an extracted epoch based on a day or a window, which pre-selects a certain timespan of the activity data. For example, certain features may operate on daily data, weekly data or larger time scales, such as two weeks. A span of a certain day, a window of duration of one week or two weeks, respectively, may be used to select the relevant time span from the time series of activity data. The activity data within said day or window may then be further analyzed in order to derive certain features.

The most important feature is the sleep duration average daily-activity score. Sleep is preferably detected using an algorithm based on different activity in segments of different length. Based on the nature of activity within these segments the sleep-like segments are divided into wear-off and sleep. Sum of periods of sleep in a day is one of the most important features. Another important feature is a motor activity during the wake hours, specifically in the 10 most active hours. The average of the activity counts within the 10-hour window for each day as well as the midtime of daily activity peaks. Other important features include, for example, intradaily variability, in particular fast changes in activity levels during a day, move of activity peak time in the 10 hour window of the most active hours, daily percentage of low and sedentary activity, sleep quality (measured in immobile minutes), activity and fragmentation of activity after wakeup and interdaily stability (similarity in consecutive days).

The logistic regression model in the variant i) may use further data which is collected in addition to activity data. For example, the further data may be selected from daylight duration, moon phase and combinations of at least two of said features.

In addition, also body temperature, pulse, blood pressure, or data on skin galvanic response may be collected as further data. Furthermore, data obtained by electroencephalography may be collected.

Concerning variant ii), it is preferred that the questions of the questionnaire are designed such that they can be answered using a scale from a given minimum value to a given maximum value, preferably being selected in the range of from -10 to 10, more preferably in the range of from 0 to 10 and in particular, the scale is from 0 to 4.

A suitable example of questions which may be answered by a patient using a scale of for example from 0 (I do not agree) to 4 (I completely agree) is given in table 1. Questions 1 to 4 relate to depression, questions 5 to 8 relate to mania and questions 9 and 10 are included to assess the ability of the patient to concentrate and focus.

(Table 1)

| No | Group | Question |
|---|---|---|
| 1 | | I feel sad, downhearted |
| 2 | | I do not enjoy anything and nothing pleases me |
| 3 | | I have no energy |
| 4 | depressive | I feel gloomy and pessimistic about the future |
| 5 | | I feel unusually great, optimistic |
| 6 | | I have excess energy |
| 7 | | My thinking is very fast, others cannot keep up with me |
| 8 | manic | I need to sleep less than usual |
| 9 | | I feel restless, tense |
| 10 | non-specific | I cannot focus |

Preferably, the analysis of mood data according to variant ii) comprises computing a probability for a depression and computing a probability for a mania.

The computing of the probability for a mania preferably comprises computing a sum of the scales assigned to the mania related questions for a time period consisting of mood data acquired for the current week and including said sum in the fixed part of the first logistic mixed effects model.

The sum of the values assigned to the mania-related questions is part of the fixed effect part of the mixed model. Further, the model preferably includes a fixed/global parameter modelling the increase of probability of mania relapse with the increase of the sum of mania related questions, a fixed/global intercept modelling the baseline probability of a relapse and random/patient-specific intercepts that adjust the model for patients.

The computing of the probability for a depression preferably comprises computing of a sum of the scales assigned to the depression related questions and non-specific questions for a time period consisting of mood data acquired for the current week and the previous week and including said sum in the fixed part of the second logistic mixed effects model.

The model preferably comprises a fixed/global parameter modelling the change in the relapse probability in relationship to the change of the sum of depression related questions in the current week. Preferably, the model further comprises a second fixed/global parameter modelling the change of relapse probability in relationship to the change of the sum of depression related questions in the previous week and a global intercept modelling the baseline probability of relapse. Further, the model includes a random/patient-specific intercept adjusting the model predictions for the patient.

Both the first logistic mixed effects model and the second logistic mixed effects model preferably use random intercepts in the random effects part.

The first and second logistic mixed effects models are preferably trained by means of a labeled training dataset comprising a ground truth. The ground truths may in particular be provided in form of a clinical scale.

Preferably, validation of the trained models is performed after training the respective model using the training dataset. This may be performed using a separate validation dataset. Especially in case only a limited amount of data is available for training and validation, a validation may be performed for example by means of leave-one-out cross-validation or by repeatedly randomly splitting the available dataset into training datasets and validation datasets.

Using the computed probabilities, a patient is preferably classified as remission if both the probability for a depression and the probability for a mania are below given depression and mania thresholds, respectively. If the patient is not classified as remission, the patient is classified as depression if the probability for a depression is larger than the probability for a mania and is classified as mania otherwise if both the probabilities for a depression and a mania are above or equal their respective thresholds and is otherwise classified as mania if the probability for a mania is above or equal the mania threshold and as depression if the probability for a depression is above or equal the depression threshold.

The respective thresholds for mania and depression are preferable set based on the receiver operating characteristic (ROC) of the respective model.

The first and second logistic mixed effects models are trained using a labeled dataset with ground truth. Suitable datasets for use with variants i) and/or ii) of the invention may be acquired by conducting a study in which patients with bipolar disorder are regularly asked to fill out questionnaires and/or are asked to wear a wearable device to record activity. In addition, all patients are observed by psychiatrists and are evaluated by means of a common clinical scale in order to establish a ground truth.

**Example datasets**

For validation and training of the machine learning models used in variants i) and ii), a study was performed which included up to 400 patients with bipolar disorder (BD) and 50 healthy controls. The participation time frame was 18 months for patients with BD and 3 months for healthy controls. The study population included men and women between 18 and 60 years old in standard clinical treatment for BD (ICD-10 diagnosis F31.). The experimental group was distributed into 3 groups: Core, Peripheral 1 (P1) and Peripheral 2 (P2).

The inclusion criteria to the study include: diagnosis and current treatment of Bipolar disorder (F31. ICD-10) (confirmed in the CORE group by an institutional psychiatrist). The age of the patients included in the study was between 18 and 60.

All study participants were instructed to wear a wearable device for monitoring activity all of the time and to complete a self-reported questionnaire according to table 1 on a weekly basis. Accordingly, the data acquired by this study is suitable for both variants i) and ii) of the present invention.

Patients in the Core group underwent an initial medical investigation at National Institution of Mental Health Czech Republic (NIMH-CZ) upon enrollment and their diagnosis of BD was either confirmed or they were moved to the P1 or P2 group. The patients were assessed by the Montgomery-Åsberg Depression Rating Scale MADRS and the Young Mania Rating Scale YMRS every month. The clinical scales were used as objective measures of depressive and manic states. Information about clinical episodes, hospitalizations and work incapacities were collected retrospectively every 6 months from the caregiving psychiatrists in all patient groups. The rating team executing the clinical scales consisted of psychologists, who went through training sessions and inter-rater reliability evaluations during the study.

**Dataset for variant i)**

Records of cases with hospitalizations were excluded from the dataset, as the activity is restricted in hospitals.

Only records having less than a defined threshold of missing activity data within a 14-day were included in the dataset. In the present example, the threshold was set to allow 10% of missing values in a 14-day window which allows for 1.4 days of missing activity data.

When features were extracted from the records based on activity data within a certain timespan defined by a day or by a window, preprocessing was performed in case of missing values. In particular, data processing was performed to fill in missing values.

In the case of features based on activity data within a given day, handling of missing data was done differently than for features based on a window. For daily based features, as these were only individual days missing, the missing values were estimated as a linear interpolation of surrounding values.

In case of week-window based features, the first seven values of these features are using unspecified data from before the annotated episode. Therefore any missing value was replaced by the average of values from the second week of the episode. In case of two-week-window based features, all, except the last value, of these features are using days from unspecified data prior to an annotated episode all missing values are replaced by the last day value of the episode.

In the following, some features which may be used are described in more detail as an example. It is to be understood that the proposed method may also be applied to different features or a different selection of the described features.

The features used may be divided by their origin:

The first group are parametric features which are obtained by fitting the cosine function of a given period (24 hours) on the raw activity data in a week or two weeks long window. The function is fitted using the least squares method. Obtained features are the offset of the fitted cosine function, and amplitude of the fitted cosine, and acrophase which is representing the phase shift of the function, and daily activity rhythm which is the amplitude normalized by mesor.

The second group is represented by nonparametric features (no specific function is used to represent the data). These features are estimated either based on individual days or an average obtained by averaging 7 or 14 consecutive days. These parameters are average activity in the 10 most active hours of the day (M10), average activity of the least active 5 hours of the day (L5), relative amplitude obtained from formula (M10-L5)/(M10+L5), ant the midtimes of both L5 and M10 windows (M10/L5 time). To describe stability of the rhythm within a day or between the consecutive days the intradaily variability (IV) and interdaily stability (IS) features were estimated. These were calculated based on a known formula from 20 to 60 minute aggregated actigraphic data segments. The formular is published in Van Someren EJ, Swaab DF, Colenda CC, Cohen W, McCall WV, Rosenquist PB. "Bright light therapy: improved sensitivity to its effects on rest-activity rhythms in Alzheimer patients by application of nonparametric methods." Chronobiol Int. 1999;16(4):505-518. http://www.ncbi.nlm.nih.gov/pubmed/10442243. Other features focus on levels of activity, the histogram of activities without sleep is divided individually into four levels (low, sedentary, moderate, high) and percentage of each level within a calendar day is calculated. Then activities are estimated in different parts of day midnight to 6AM, 6AM to noon and so on (QA1-QA4), special focus is given the time after wakeup and before sleep onset (the average activity in a two hour window).

The third group are sleep describing features. These are duration of the main sleep of the day, as well as all sleeps in a given day (18 o'clock previous day till 18 o'clock actual day). Based on the main daily sleep the sleep quality is detected using number of immobile minutes (minutes where almost no movement is detectable < 20 mg {after filtering}), the midtime of the main sleep, Wake after sleep onset (number of minutes that are not detected as sleep during the main sleep of a day). To include part of the year and approximate night illumination features of daylight duration and moon phase are also included in the models.

In a final dataset used for variant i), there were 56 depression episodes, 25 mania episodes, and 172 remission episodes, which were analyzed. Multiple episodes of one type may be found for some patients. As the models are trained solely on patients with episodes in both tested classes, there were 10 patients with both depression and mania, 15 patients with both remission and mania and 28 patients with remission and depression.

### Dataset for variant ii)

Matching of the clinical scales and the self-report questionnaires was performed based on the date of the event of the filling-in of the questionnaire and the date of the structured interview for the assessment of the clinical scales. If the difference between these dates was lower than a threshold value, the clinical scale and the self-report were considered matching. In the present example, the threshold was set to 3 days for questionnaires relating to the present situation (zero delay). For questionnaires relating to the previous week, the threshold was set to 10 days and for questionnaires relating to the two weeks before the clinical scale, the threshold was set to 17 days. All the matchings were unique, none of the clinical scales was matched to more than one of the self-reports and vice versa for each delay. In order to exploit the dependencies in the consecutive questionnaires, the data from more than one week preceding the clinical scale were considered.

Only patients that had both relapse and remission according to the clinical scales were included in the obtained dataset. A relapse according to the clinical scales is defined as $\Sigma$MADRS >= 15 in case of depression and $\Sigma$YMRS >= 15 in case of mania. Only complete records were included in the dataset.

Concerning depression according to the MADRS scale, the dataset includes records of 67 patients with 1264 paired observations for the concurrent clinical scales and self-report, 1159 observations from the previous week and 11180 two weeks before. From the 1264 observations there are 251 relapses and 1013 relapses.

Concerning mania according to the YMRS scale, the dataset includes records of 31 patients represented by 614 paired observations for the concurrent clinical scales and self-reports, 560 observations from the previous week and 563 observations two weeks before. From the 614 clinical scales there are only 74 relapses and 540 relapses.

### Training results using the example dataset

### Analysis of activity data according to variant i)

An example Depression-Mania model is based on cosine of the amplitude, from non-parametric L5, L5 time, M10, RA, daily part of sedentary activity, IV, IS, Average activity during morning and evening (QA2, QA4), based on sleep the immobile minutes (percentage), sleep midtime. The model also uses a year season represented by daylight duration. The most important features are L5, amount of sedentary activity and IV.

An example Depression-Remission model is based on cosine acrophase, mesor and amplitude, based on non-parametric analysis IS, IV, M10, M10 time, L5, L5 time, before and after sleep average activities, daily part of moderate and low activities, and activity during morning hours (QA2), based on sleep the WASO, and sleep duration. The strongest predictors are acrophase, IS, IV, M10 and activity before sleep

An example Mania-Remission model uses from cosine analysis acrophase, and mesor, from non-parametric analysis L5 time, L5, M10 time, IV, IS, night and morning activity (QA1, QA2), DAR, RMSSD, and daily percentage of sedentary and low activity, based on sleep percentage of immobile minutes, main sleep duration, and daily (overall) sleep duration. The model also uses a year season represented by daylight duration. The most important are immobile minutes, L5 time, IV, and M10 time.

Two models of each type based on logistic regression were trained using the example dataset. A first model includes values computed using features based on the evaluation of motor activity data of a certain time window, for example a day. A second model is based on difference values of the respective features between two time periods (these are the same features as in the first model, only in this case it's differences from individual patients mean values). The results for the two models are given in table 2.

The features for the respective models are selected separately based on the prediction model. The features calculated for each day (end of window) are used in the model to distinguish between states and the final output (classification) is obtained based on the combined results of the models.

(Table 2)

|  | Dep-man | Dep-rem | Man-rem |
|---|---|---|---|
| Logistic Regression | Accuracy 0.61<br>Sensitivity 0.65<br>Specificity 0.55<br>AUC: 0.71# | Accuracy 0.61<br>Sensitivity 0.60<br>Specificity 0.52<br>AUC: 0.58# | Accuracy 0.61<br>Sensitivity 0.49<br>Specificity 0.64<br>AUC: 0.70# |
| Logistic Regression (diff features) | **Accuracy 0.66**<br>**Sensitivity 0.70**<br>**Specificity 0.64**<br>AUC: 0.66# | Accuracy 0.57<br>Sensitivity 0.61<br>Specificity 0.51<br>AUC: 0.56# | Accuracy 0.60<br>Sensitivity 0.44<br>Specificity 0.71<br>AUC:0.70# |

In the table, AUC denotes the area under the receiver operating characteristic curve (ROC curve) and is an indication of the performance of the classification model. A value of 0 indicates 100% wrong classification and a value of 1 indicates a 100% correct classification.

**Analysis of mood data according to variant ii)**

In the present example, the first logistic mixed effects model uses a sum of the scales assigned to the mania related questions for a time period consisting of mood data acquired for the current week in the fixed part of the model.
The second logistic mixed effects model uses a sum of the scales assigned to the depression related questions and non-specific questions for a time period consisting of mood data acquired for the current week and the previous week in the fixed part of the model.
Both the first logistic mixed effects model and the second logistic mixed effects model preferably use random intercepts in the random effects part.
For training of the first and second logistic mixed effects models, the dataset was split into training and testing data. The proportions were 70% of the data for training and 30% for testing. 999 sets of training and testing data were randomly chosen from the dataset. The models were trained on the training sets; the training sets predictions and true values of the scale based relapses were used to construct a receiver operating characteristic (ROC), which was used to estimate the threshold probability maintaining 0.9 specificity to reduce the number of false alarms. The model prediction was modified to maintain specificity 0.9 by altering the threshold probability according to the ROC curve from training data prediction.
In the case of the first logistic mixed effects model for assessing mania, the validation results for the trained model are summarized in Table 3 below:

(Table 3)

| Set | Accuracy | AUC | Sensitivity | Specificity |
|---|---|---|---|---|
| training | 0.886 | 0.916 | 0.760 | 0.903 |
| testing | 0.875 | 0.871 | 0.711 | 0.897 |

In the case of the second logistic mixed effects model for assessing depression, the validation results for the trained model are summarized in Table 4 below

(Table 4)

| Set | Accuracy | AUC | Sensitivity | Specificity |
|---|---|---|---|---|
| training | 0.883 | 0.931 | 0.807 | 0.902 |
| testing | 0.849 | 0.878 | 0.717 | 0.880 |

The presented results show the predictive ability of the self-report questionnaire in scale-based relapse prediction. The area under the ROC curve (AUC) is 0.871 in the case of the first model (mania) and is 0.878 in the case of the second model (depression) on 999 randomly chosen testing data sets. The value of AUC is less sensitive to the class imbalance than the accuracy. The class imbalance is rather high in both depression and mania (only 19.8% events represent relapses for depression and 12.0% for mania) and using only accuracy, it would be possible to obtain better results just by assigning all the events as remission (appr. 0.80 and 0.88 for depression and mania respectively for the case of assigning all events as remission; in comparison to the actual observed 0.85 and 0.88 for depression and mania respectively). The relatively high values of the sensitivity in both cases indicate that the model is actually correctly predicting the relapses.

Preferably, the design of the questions of the questionnaire of variant ii) is validated by using principal component analysis and a test dataset which includes ground truth.

Principal component analysis is a method for analyzing the relation of data and for feature extraction. The method involves an orthogonal linear transformation that transforms the data to a new coordinate system defined by the eigenvectors. The transformation is performed such that the greatest variance of a scalar projection of the data comes to lie on the first coordinate. The second largest variant comes to lie on the second coordinate and so on.

The mania related questions being assigned to the same principal component would indicate that the questions are related. Likewise, the depression related questions being assigned to the same principal component would indicate that the respective questions are related. Still further, if questions relating to mania as well as questions relating to depression would be assigned to the same principal component this would indicate a low selectivity. Accordingly, the two major principal components should only include questions relating to mania or relating to depression, respectively.

Tables 5 and 6 present the results of the PCA analysis performed on the example dataset. There are two dominant components (self report pc1 50.3%, pc2 22.1%). The first principal component pc1 relates to the depression-related questions (pc1 high values of loadings for questions 1-4) and to the non-specific questions (pc1 high values of loadings for questions 9 and 10). The second principal component pc2 summarizes the mania-related questions (pc2 high values of loadings for questions 5-8), the non-specific questions (pc2 high values of loadings for questions 9 and 10), but their loading coefficients are smaller than those of the main mania-related questions. All the remaining principal components pc3 to pc10 explain less than 7% variability each.

The results of the principal component analysis indicate that the questions presented in table 1 are suitable as they relate to depression and mania, respectively, as intended.

(Table 5)

|  | PC1 | PC2 | PC3 | PC4 | PC5 | PC6 | PC7 | PC8 | PC9 | PC10 |
|---|---|---|---|---|---|---|---|---|---|---|
| **Q 1** | 0.428 |  | 0.281 | -0.272 |  | -0.348 |  | -0.44 | -0.57 | 0.129 |
| **Q 2** | 0.43 |  | 0.304 | -0.14 |  |  |  | -0.282 | 0.716 | -0.311 |
| **Q 3** | 0.42 | -0.118 | 0.202 | 0.721 | 0.427 |  |  | 0.165 |  | 0.178 |
| **Q 4** | 0.396 |  | 0.237 | -0.34 | -0.286 | 0.212 | -0.109 | 0.719 | -0.103 |  |
| **Q 5** |  | 0.502 | 0.271 | 0.364 | -0.358 | -0.157 | -0.376 |  | -0.164 | -0.468 |
| **Q 6** |  | 0.492 | 0.225 |  | -0.164 |  |  | -0.103 | 0.284 | 0.761 |
| **Q 7** |  | 0.425 | 0.156 |  |  | 0.116 | 0.853 |  |  | -0.183 |
| **Q 8** |  | 0.432 |  | -0.367 | 0.727 | 0.15 | -0.323 |  |  | -0.139 |
| **Q 9** | 0.366 | 0.259 | -0.608 |  |  | -0.597 |  | 0.225 | 0.134 |  |
| **Q10** | 0.399 | 0.208 | -0.46 |  | -0.216 | 0.639 |  | -0.333 | -0.116 |  |

(Table 6)

|  | PC1 | PC2 | PC3 | PC4 | PC5 | PC6 | PC7 | PC8 | PC9 | PC10 |
|---|---|---|---|---|---|---|---|---|---|---|
| **Standard deviation** | 1.416 | 0.938 | 0.529 | 0.422 | 0.401 | 0.348 | 0.333 | 0.326 | 0.274 | 0.269 |
| **Proportion of variance** | 0.503 | 0.221 | 0.07 | 0.045 | 0.04 | 0.03 | 0.028 | 0.027 | 0.019 | 0.018 |
| **Cumulative proportion of variance** | 0.503 | 0.723 | 0.794 | 0.838 | 0.878 | 0.909 | 0.936 | 0.963 | 0.982 | 1 |

For further verification of the selection of the questions so that the obtained results correspond to an established clinical scale, mood data according to variant ii) is preferably analyzed using a linear mixed effects model utilizing a time series of obtained mood data.

Mixed linear effects models may be used in binary classification problems and comprise fixed and random effects. In a first mixed effects linear model describing the probability for the patient being in the state of remission or mania, the mania related questions are included as a linear combination. In a second mixed effects linear model describing the probability for the patient being in the state of remission or depression, the depression related questions are included as a linear combination.

The first model uses the scales assigned to the mania-related questions as a fixed effect and includes random slopes and random intercepts for the sum of mania-related questions for each patient.

Likewise, the second model uses the scales assigned to the depression-related questions as fixed effect and includes random slopes and random intercepts for the sum of depression-related questions for each patient.

Preferably, the machine learning model(s) used for evaluation of activity data according to variant i) is trained and/or verified against clinical classification scales.

Suitable clinical scales for validating the questionnaire according to variant i) and/or for verifying the model(s) according to variant ii) may, for example, be selected from the Clinical Global Impression scale (CGI) to characterize the severity of a bipolar disorder, the MADRS scale, Bipolar Depression Rating Scale (BDRS) in case of depression and the YMRS scale, Observer-Rated Scale for Mania (IRSM) or the Bech-Rafaelsen Mania Rating Scale (MAS) in case of mania. The clinical scale may be selected independently for each of the variants i) and ii) and for each of the model(s) used in any of the variants i) and ii).

The invention further relates to a method for treating a patient having bipolar disorder wherein i) activity data is recorded using a wearable device worn by the patient and is input into an evaluating system and/or ii) mood data is obtained by obtaining a questionnaire which has been completed by the patient, the questions of the questionnaire relating to the mania state, to the depression state and further include at least one control question for checking the awareness and/or the ability to focus of the patient, the questions being designed such that they can be answered by multiple choice, and the answers of the patient are input into the evaluating system, the evaluating system analyzes the input data by means of machine learning, wherein the condition of the patient is classified as remission, mania or depression, and wherein a relapse is detected if the patient is classified as mania or depression. Further, in case a relapse is detected, therapeutic intervention may be performed.

In case a therapeutic intervention is needed, the physician may, after he/she may have discussed the possible consequences with the patient, take the appropriate measures depending on the disease. This may include the administration or the adjustment of the medication needed for treating the patient's disease, other measures like psychotherapy, light therapy, rTMS (Repetitive Transcranial Magnetic Stimulation) or physical therapy as well as surgery. Administration of medication may include antipsychotics and mood stabilizers e.g. based on lithium/valproate like lurasidone, olanzapine and quetiapine.

It is a further object of the invention to provide an evaluating system for detection of a relapse into a depression or mania state of a patient in a remission state based on activity data and/or mood data. The evaluating system comprises an evaluating unit which is configured to analyze activity data and/or mood data according to any one of the methods described herein.

Preferably, the system comprises at least one wearable device for obtaining activity data which is configured to periodically transmit recorded time series of activity data to the evaluating unit. The wearable device is preferably configured as a wristband or wristwatch.

The system may further comprise an interface device configured to receive activity data and/or mood data from other devices, in particular user devices such as smartphones or tablets.

The invention is not limited to the exemplary embodiments described herein and the aspects highlighted therein. Rather, within the range indicated by the claims, a large number of variations are possible.

## Claims

1. Method for detection of a relapse into a depression or mania state of a patient from a remission state wherein

   i) motor activity data is recorded using a wearable device worn by the patient and is received as input data by an evaluating unit and/or

   ii) mood data is acquired by obtaining a questionnaire which has been completed by the patient, the questions of the questionnaire relating to the mania state, to the depression state and the questionnaire including at least one control question for checking the awareness and/or the ability to focus of the patient, the questions being designed such that they can be answered by multiple choice, and wherein the answers of the patient are input as input data into the evaluating unit,

the input data is analyzed by the evaluating unit, wherein the condition of the patient is classified as remission, mania or depression by means of machine learning, and
wherein a relapse is detected if the patient is classified as mania or depression.

2. Method according to claim 1, wherein motor activity data according to variant i) is analyzed using logistic regression method utilizing a time series of motor activity data and/or wherein mood data according to variant ii) is analyzed using logistic mixed effects models utilizing a time series of obtained mood data.

3. Method according to claim 1 or 2, wherein the machine learning model used according to variant i) comprises binary classification models for classification of remission/mania, depression/mania and remission/depression and for classification into one of the three classes remission, mania and depression

   a) a score is computed for each of the three classes based on probabilities obtained from the binary classification models, or
   b) one of the three classes is selected based on majority voting where two of the three classification models indicate the same class.

4. Method according to claim 2 or 3, wherein features are extracted from the time series of motor activity data and the extracted features are used in the logistic regression model, the features being selected from sleep features, in particular sleep duration, activity during sleep and fragmentation of sleep, activity distribution within the day, in particular the amount of activity in the active part of a day, overall activity level, fragmentation of activity within a day, the timing of activities throughout a day and combinations of at least two of said features.

5. Method according to any one of claims 1 to 4, wherein in variant i) further data is collected in addition to motor activity data, wherein the further data is selected from daylight duration, moon phase, body temperature, pulse, blood pressure, data on skin galvanic response and combinations of at least two of said features.

6. Method according to any of one claims 1 to 5, wherein the time series of motor activity data according to variant i) is pre-processed to extract epochs which represent motor activity data over a time window of individual days or weeks or represent the time of night-sleep.

7. Method according to any one of claims 1 to 6, wherein the questions according to variant ii) are designed such that they can be answered using a scale from a given minimum value to a given maximum value, preferably being selected in the range of from -10 to 10, more preferably in the range of from 0 to 10 and in particular, the scale is from 0 to 4.

8. Method according to claim 7, wherein the analysis of mood data according to variant ii) comprises computing a probability for a depression and computing a probability for a mania.

9. Method according claims 2 and 7 and 8, wherein computing of the probability for a depression comprises computing of a sum of the scales assigned the depression related questions and non-specific questions for a time period consisting of mood data acquired for the current week and the previous week and including said sum in the fixed part of the linear mixed effects model.

10. Method according to claims 2 and 7 and 8, wherein computing of the probability for a mania comprises computing of a sum of the scales assigned the mania related questions for a time period consisting of mood data acquired for the current week and including said sum in the fixed part of the linear mixed effects model.

11. Method according to any one of claims 7 to 10, wherein a patient is classified as remission if both the probability for a depression and the probability for a mania are below given depression and mania thresholds, respectively, and, if the patient is not classified as remission, the patient is classified as depression if the probability for a depression is larger than the probability for a mania and is classified as mania otherwise if both the probability for a depression and probability for a mania are above or equal their respective thresholds and is otherwise classified as mania if the probability for a mania is above or equal the mania threshold and as depression if the probability for a depression is above or equal the depression threshold.

12. Method according to any one of claims 1 to 11, wherein the design of the questions of the questionnaire of variant ii) is validated by using principal component analysis and a test dataset which includes ground truth.

13. Method according to any one of claims 1 to 12, wherein the machine learning model(s) used for evaluation of motor activity data according to variant i) is trained and/or verified against clinical classification scales.

14. Method according to any one of claims 1 to 13, wherein the clinical scale for validating the questionnaire according to variant i) and/or for verifying the model(s) according to variant ii) is the Clinical Global Impression scale (CGI) to characterize the severity of a bipolar disorder, the MADRS scale, Bipolar Depression Rating Scale (BDRS) in case of depression and the YMRS scale, Observer-Rated Scale for Mania (IRSM) or the Bech-Rafaelsen Mania Rating Scale (MAS) in case of mania.

15. Evaluating system for detection of a relapse into a depression or mania state of a patient in a remission state based on motor activity data and/or mood data, **characterized in that** the evaluating system comprises an evaluating unit which is configured to analyze motor activity data and/or mood data according to a method of any one of claims 1 to 14.

Europäisches
Patentamt
European
Patent Office
Office européen
des brevets

## EUROPEAN SEARCH REPORT

Application Number

EP 20 19 1902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/117143 A1 (FEDOR SZYMON [US] ET AL) 25 April 2019 (2019-04-25) <br> * abstract * <br> * paragraphs [0004], [0005], [0011], [0027], [0031], [0041] - [0046], [0068], [0072], [0076] - [0079], [0083] - [0084], [0090], [0121]; claim 1; figures 3A-B, 4 * | 1,2,4-15 | INV.<br>A61B5/16<br>A61B5/11<br><br>ADD.<br>A61B5/00 |
| X | MAXHUNI ALBAN ET AL: "Classification of bipolar disorder episodes based on analysis of voice and motor activity of patients", <br> PERVASIVE AND MOBILE COMPUTING, ELSEVIER, NL, <br> vol. 31, 29 January 2016 (2016-01-29), pages 50-66, XP029750573, <br> ISSN: 1574-1192, DOI: <br> 10.1016/J.PMCJ.2016.01.008 <br> * abstract * <br> * sect.3.2, 4.1-4.3, 5.1, 5.3,5.4; table 10 * | 1-4, 12-14 | |
| X,D<br><br>A | EP 3 430 978 A1 (MINDPAX S R O [CZ]) 23 January 2019 (2019-01-23) <br> * abstract * <br> * claims 1-7 * | 1,15<br><br>2,14 | TECHNICAL FIELDS SEARCHED (IPC)<br><br>A61B<br>G16H |
| A | ZHONGDE PAN ET AL: "Detecting Manic State of Bipolar Disorder Based on Support Vector Machine and Gaussian Mixture Model Using Spontaneous Speech", <br> PSYCHIATRY INVESTIGATION, <br> vol. 15, no. 7, 25 July 2018 (2018-07-25), pages 695-700, XP055755183, <br> ISSN: 1738-3684, DOI: <br> 10.30773/pi.2017.12.15 <br> * the whole document * | 1-15 | |

-/--

The present search report has been drawn up for all claims

| Place of search | Date of completion of the search | Examiner |
|---|---|---|
| The Hague | 30 November 2020 | Delval, Christophe |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches
Patentamt

European
Patent Office

Office européen
des brevets

**EUROPEAN SEARCH REPORT**

Application Number

EP 20 19 1902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 106 725 532 B (UNIV LANZHOU) 24 April 2018 (2018-04-24) * abstract * * claims 1,2 * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 30 November 2020 | Delval, Christophe |

## ANNEX TO THE EUROPEAN SEARCH REPORT
## ON EUROPEAN PATENT APPLICATION NO.

EP 20 19 1902

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2020

| Patent document cited in search report | | Publication date | Patent family member(s) | Publication date |
|---|---|---|---|---|
| US 2019117143 | A1 | 25-04-2019 | NONE | |
| EP 3430978 | A1 | 23-01-2019 | NONE | |
| CN 106725532 | B | 24-04-2018 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• EP 3430978 A

**Non-patent literature cited in the description**

• **VAN SOMEREN EJ ; SWAAB DF ; COLENDA CC ; COHEN W ; MCCALL WV ; ROSENQUIST PB.** Bright light therapy: improved sensitivity to its effects on rest-activity rhythms in Alzheimer patients by application of nonparametric methods. *Chronobiol Int.,* 1999, vol. 16 (4), 505-518, http://www.ncbi.nlm.nih.gov/pubmed/10442243